# FASCICULE DE BREVET EUROPEEN

(11) **EP 0 469 952 B1**
(45) Date de publication et mention de la délivrance du brevet: **03.05.2000**
(21) Numéro de dépôt: 91402017.7
(22) Date de dépôt: 19.07.1991
(51) Int. Cl.: C12N 1/16, C12N 11/08, A23B 4/12, A23B 4/22, A23B 4/10, C12R 1/645

(54) **Nouveaux microorganismes et leur utilisation pour la fabrication d'articles de charcuterie**
Neue Mikroorganismen und ihre Verwendung zur Herstellung von Fleischwarenartikeln
Novel microorganisms and their use in the production of meat products

(30) Priorité: 30.07.1990 FR 9009668
(43) Date de publication de la demande: 05.02.1992
(73) Titulaire: TEXEL, 86220 Dange-Saint-Romain (FR)
(72) Inventeur: Leroy, Marie-Josée, Dreieichenhain (DE); Guerineau, Pierre, F-86580 Biard (FR)
(74) Mandataire: Fabre, Madeleine-France

(56) Documents cités:
- US-A- 4 886 673
- RTVA vol. 18, no. 52, Octobre 1979, page 50; Y. VAYSSIER: "MISE AU POINT D'UNE NOUVELLE MICROFLORE DE SURFACE POUR LE FLEURAGE DU SAUCISSON SEC"

## Description

La présente invention concerne de nouveaux microorganismes qui permettent notamment de former une fleur de surface sur la peau d'articles de charcuterie tout en inhibant la formation d'une flore contaminante.
L'invention concerne également l'utilisation de ces microorganismes pour la fabrication d'articles de charcuterie.

Il est connu que des microorganismes tels que des levures ou des moisissures, en particulier des levures appartenant au genre Debaryomyces et des moisissures appartenant au genre Penicillium, peuvent être utilisés pour la formation de fleur de surface sur la peau d'articles de charcuterie, notamment des saucisses et saucissons. En particulier, l'article (RTVA, vol. 18, n^{º} 52, Octobre 1979, page 50) de Y. Vayssier "Mise au point d'une nouvelle microflore de surface pour le fleurage du saucisson sec" rappelle l'utilisation de Debaryomyces hansenii et Penicillium nalgiovense comme agent de fleurage et d'arôme.

La fleur de surface a des actions protéolytiques et lipolytiques et permet par ces actions de renforcer le goût et l'arôme de l'article de charcuterie.
De plus, par son aspect, la fleur de surface doit rendre l'article de charcuterie plus attrayant et plus agréable "à l'oeil". Enfin, la fleur de surface permet de réguler la perte en eau de l'article de charcuterie au cours de sa fabrication.

Toutefois avec les microorganismes utilisés jusqu'à présent, l'aspect de la fleur de surface est imparfait. Ainsi, les fleurs de surface connues jaunissent au cours du temps. De ce fait, il est souvent nécessaire d'éliminer la fleur de surface de l'article de charcuterie une fois qu'elle a accompli ses actions protéolytiques et lipolytiques, ceci au cours d'une opération dite de brossage. Suite au brossage il est alors courant lors d'une opération dite de poudrage, de rajouter du talc ou de la farine sur la peau de l'article de charcuterie, afin de lui procurer un aspect de surface blanc poudreux.

Bien entendu, ces opérations de brossage et de poudrage constituent une perte de temps et donc de productivité pour les fabricants d'articles de charcuterie.

D'autre part, les fleurs de surface obtenues avec lesdits microorganismes connus présentent une mauvaise adhérence à la peau de l'article de charcuterie.
Enfin, les fleurs de surface classiques au cours de la conservation de l'article de charcuterie, sont contaminées par des flores indésirables. Ces contaminants se présentent généralement sous la forme de taches de couleur à la surface de l'article de charcuterie. Ces taches gâchent l'aspect et même le goût de l'article de charcuterie. Elles se forment au bout d'environ 10 à 15 jours après l'ensemencement du microorganisme sur l'article de charcuterie.

Il existe donc un grand besoin dans l'industrie de la charcuterie pour des microorganismes permettant d'obvier les inconvénients de l'art antérieur et surtout d'inhiber la formation de la flore contaminante.
Au cours de ces recherches la demanderesse a isolé un microorganisme qui permet de former une fleur de surface de couleur crème, quine jaunit pas au cours du temps, dont l'aspect est ras et poudreux, non poisseux, qui évite le brossage et un éventuel poudrage et surtout qui empêche la formation de la flore contaminante.
De plus, la fleur de surface ainsi formée adhère bien à la peau de l'article de charcuterie. Enfin, le dit microorganisme permet une amélioration notable du goût et de l'arôme dudit article de charcuterie.
L'invention concerne donc un microorganisme caractérisé en ce qu'il est notamment adapté pour la formation de fleur de surface sur des peaux d'articles de charcuterie et qu'il permet d'inhiber la formation de la flore contaminante de ladite fleur de surface.
Ledit microorganisme est une levure appartenant à l'espèce Debaryomyces hansenii et est choisie parmi la souche CBS 291.90 ou parmi ceux de ses mutants ou recombinants qui présentent la propriété d'inhiber la formation de flore contaminant d'article de charcuterie et de former une fleur de surface sur la peau des articles de charcuterie.
Dans un cadre particulièrement préféré de la présente invention ledit microorganisme présente les propriétés d'inhibition de la flore contaminante de la fleur de surface de la souche Debaryomyces hansenii CBS. 291.90.
Cette souche a été déposée auprès du Centraalbureau Voor Schimmelcultures (CBS) dans le cadre du Traité de Budapest, le 12 Juillet 1990.
Bien entendu, ledit microorganisme peut se présenter sous la forme d'une culture pure. Généralement la flore contaminante dont la formation est inhibée par le microorganisme objet de l'invention comporte ou est généralement constituée de moisissures appartenant au groupe des Mucorales, tels Mucor circinelloïdes ou Mucor plumberus, au groupe des Ascomycetes, tels Eurotium echinulatum, Eurotium herbariorum, Eurotium amstelodami ou Eurotium chevalieri, ou au groupe des Deuteromycetes tels Aspergillus groupe glaucus, Aspergillus niger, Cladosporium herbarum, Fusarium, culmorum, Gliocladium roseum, Penicillium brevicompactum, Penicillium corylophilum, Penicillium cyclopium, Penicillium decumbens, Penicillium digitatum, Penicillium-expansum, Penicillium glabrum, Penicillum griseofulvum, Penicillium janthinellum, Phoma glomerata, Scopulariopsis brevicaulis, Trichodenma koningii, Trichotecium roseum ou Wallemia sebi.

Les moisissures appartenant au genre Penicillium sont les plus fréquentes.

Selon la nature de ces microorganismes, ils forment des taches de couleur différente et conventionnellement appelées "bleu", "vert", "jaune" ou "gris". De plus, il a été constaté que d'une manière surprenante, le microorganisme selon l'invention permet de réduire considérablement le nombre de bactéries pathogènes, tels des Coliformes et surtout des Entérocoques, des Micrococcaceae ou des bactéries psychrotrophes qui peuvent apparaître sur la peau d'un article de charcuterie. Le microorganisme selon l'invention permet une inhibition de la flore contaminante sur une période au moins égale à un mois, généralement supérieure à 3 mois, lorsque l'article de charcuterie est conservé à l'air ambiant. Lesdits articles de charcuterie sont de préférence des saucisses ou des saucissons à base de viande, généralement d'ovins, de bovins, d'équinés et/ou de porcins, recouverts d'une peau qui peut être soit un boyau naturel tels un menu, utilisé principalement pour les saucisses sèches de type "bâton", un chaudin, utilisé pour les saucissons ménages ou rosettes, soit un boyau synthétique de nature cellulosique ou collagénique utilisé principalement pour saucissons d'Arles et salamis et dont le diamètre peut être compris entre 50 et 130 mm.
Le menu provient généralement de l'intestin grèle des ovins, des porcins, des équinés ou des bovins. Son diamètre peut être compris entre 15 et 80 mm.
Le chaudin provient habituellement du gros intestin des porcins. Son diamètre peut être compris entre 40 et 80 mm.

Lesdits articles de charcuterie peuvent être également des jambons ou des produits type saucisse à base de poissons ou de légumes fermentés, en mélange ou non avec des viandes, et recouverts d'une peau.
Ces viandes, poissons et/ou légumes peuvent être fermentés avec des ferments de maturation classiquement utilisés en charcuterie.
Un autre aspect de l'invention concerne une levure dont les caractéristiques d'identification sont essentiellement celles de ladite souche Debaryomyces hansenii CBS 291.90, ainsi qu'une levure qui est Debaryomyces hansenii CBS 291.90 un de ses mutants ou un de ses recombinants.
Bien entendu ladite levure peut se présenter sous la forme d'une culture pure.
Les caractéristiques taxonomiques de la souche Debaryomyces hansenii CBS 291.10, connues de la demanderesse, sont les suivantes :
1. Caractéristiques morphologiques :
   a) bourgeonnement des cellules,
   b) pas de formation d'arthroconidies, d'endospores, de chlamydospores ou de ballistoconidies,
   c) les asques sont non évanescents.
2. Caractéristiques physiologiques et biochimiques :
   - a) souche mésophile:
   - b) résistante à 0,01 % de cycloheximide:
   - c) production d'acide acétique: négatif
   - d) production d'éthanol: négatif
   - e) croissance sur ::
   - D-glucose: positif
   - D-galactose: positif
   - L-sorbose: positif
   - D-glucosamine: négatif
   - D-ribose: négatif
   - D-xylose: positif
   - L-arabinose: positif
   - L-rhamnose: positif
   - saccharose: positif
   - maltose: positif
   - α,α tréhalose: positif
   - melézitose: positif
   - meliliose: négatif
   - méthylα - glucoside: positif
   - cellobiose: positif
   - lactose: négatif
   - raffinose: positif
   - glycérol: positif
   - meso erythritol: positif
   - D-glucitol: positif
   - D-mannitol: positif
   - myo-inositol: négatif
   - 2-Céto-D-gluconate: positif
   - D-gluconate: positif
   - D-glucuronate: négatif
   - DL - lactate: positif
   - nitrate: négatif
   - éthylamine: positif
   - L- lysine: positif
   - cadaverine: positif
   - f) fermentation sur ::
   - glucose: négatif
   - galactose: négatif
   - maltose: négatif
   - saccharose: négatif
   - lactose: négatif
   - raffinose: négatif

La fabrication de la souche Debaryomyces hansenii CBS 291.90 peut se faire par la préparation, dans un premier temps, d'un inoculum et dans un second temps par culture d'un milieu de fermentation ensemencé par l'inoculum.
Cet inoculum peut être obtenu par ensemencement d'un milieu dont la composition peut être identique à celle d'un milieu de fermentation tel que décrit ci-dessous.

Des quantités plus importantes de microorganismes peuvent être obtenues par ensemencement d'un milieu de fermentation par un tel inoculum.
Un milieu de fermentation adéquat peut comporter au moins une source carbonée et au moins une source azotée assimilable par ladite souche.
A titre de source carbonée, on préfère les hydrates de carbone parmi lesquels on peut citer le galactose, le sorbose, le maltose, le dextrose, l'amidon, les hydrolysats d'amidon.
Le dextrose est une source carbonée préférée.
A titre de source azotée, on peut utiliser une source azotée organique en mélange ou non avec une source azotée minérale.
A titre de source azotée organique, on peut citer la tryptone, les extraits de levures, en particulier les levures de boulanger, la levure de bière, les levures lactiques, le corn steep liquor, les farines de poisson, les caséines et les caséinates.
Un mélange de tryptone et d'extraits de levures est préférée. Si on utilise un tel mélange, le rapport entre la tryptone et les extraits de levure est généralement compris entre 10/90 à 90/10.
Génératement la concentration de la source carbonée dans le milieu de fermentation est comprise entre 5 et 70 g/l, de préférence entre 15 et 40 g/l.
En outre le milieu de fermentation peut comporter des sels minéraux de préférence des sels de phosphates, de magnésium et de manganèse, en particulier des sels alcalins, alcalino-terreux ou ammoniaqués de phosphates comme le diammonium dihydrogénophosphate ainsi que les sulfates de manganèse et de magnésium.
La concentration desdits sels minéraux dans le milieu de fermentation est généralement comprise entre 0,1 à 4 g/l, de préférence entre 1 et 3 g/l.
Enfin, le milieu de fermentation peut comprendre des adjuvants classiquement utilisés dans un tel milieu, comme par exemple des antimousses et des antioxydants.
Au cours de la fabrication de la souche Debaryomyces hansenii CBS 291.90 par fermentation d'un milieu tel que décrit ci-dessus, la température est généralement réglée entre 20 et 30°C.
Le pH peut être ajusté au départ entre 5 et 7 et être maintenu entre de telles valeurs pendant la fermentation.
A la fin d'une opération de fabrication du microorganisme, c'est-à-dire généralement plus de 30 heures, de préférence entre 30 et 40 heures, après l'ensemencement du milieu de fermentation, on refroidit éventuellement le milieu de fermentation à une température de l'ordre de 20°C, puis on récupère les microorganismes fabriqués.
La récupération du microorganisme peut se faire par tout moyen physique de séparation classique qui permet de séparer substantiellement le milieu de fermentation des microorganismes. Des moyens de séparation préférés sont la filtration et plus préférentiellement la centrifugation.
Suite à la récupération des microorganismes, ces derniers peuvent être conditionnés de manière classique de sorte à pouvoir être conservés sur une longue période de temps. Ainsi lesdits microorganismes peuvent être congelés ou lyophilisés. La lyophilisation est réalisée en présence de supports classiques de lyophilisation, comme le lait, le saccharose, le lactose ou l'acide glutaminique.
Une souche Debaryomyces hansenii CBS 291.90, une fois lyophilisée peut être maintenue sans perte de viabilité pendant plus de 6 mois à 4°C ou pendant plus d'un an à -25°C.
Un autre objet de la présente invention est constitué par une association entre les microorganismes décrits ci-dessus avec au moins un microorganisme autre dont l'une des caractéristiques est de pouvoir former une fleur de surface sur la peau d'un article de charcuterie. Ledit microorganisme autre peut être une moisissure, de préférence une moisissure appartenant au genre Penicillium. Les espèces Penicillium nalgiovensis, Penicillium chrysogenum et Penicillium candidum sont plus particulièrement préférées.
Une telle association peut être constituée de 5 à 95% de microorganismes faisant l'objet de la présente invention, tel Debaryomyces hansenii CBS. 291.90. et de 95 à 5% d'au moins un microorganisme autre.

La présente invention concerne également l'utilisation desdits microorganismes ou associations décrits ci-dessus pour la fabrication d'articles de charcuterie et de préférence d'articles de charcuterie recouverts d'une peau.
Des articles de charcuterie comportant une peau recouverte d'une fleur de surface constituée par un microorganisme ou une association tels que décrits ci-dessus, font également partie de la présente invention. Ces articles de charcuterie sont de préférence des saucisses et des saucissons, généralement à base de viande, mais peuvent être constitués également d'articles de charcuterie tels ceux décrits plus haut et qui peuvent notamment comporter ou être à base de poisson ou de légumes fermentés.

Ces articles de charcuterie peuvent être notamment préparés selon un procédé consistant à ensemencer, notamment en trempant, douchant ou pulvérisant un article de charcuterie premier, c'est-à-dire non recouvert d'une fleur de surface, dans ou au moyen d'une suspension aqueuse desdits microorganismes, levure ou association puis, éventuellement, à étuver et/ou à sécher ledit article de charcuterie.
Ladite suspension aqueuse peut en outre comporter au moins un sel comestible, tel le chlorure de sodium et/ou au moins un acide organique comestible tel l'acide lactique.

Le trempage est une opération classique en charcuterie qui consiste simplement à tremper l'article de charcuterie dans ladite suspension aqueuse. Le douchage, connu en lui-même, consiste à asperger l'article de charcuterie avec ladite suspension aqueuse et à recycler cette dernière.

Pour le trempage et le douchage, on utilise généralement des suspensions comportant de 1.10¹⁰ à 5.10¹² microorganismes pour 50 litres d'eau et éventuellement de 10 à 150 g/l d'un sel comestible et jusqu'à 1 g/l dudit acide organique comestible.

Pour la pulvérisation, on utilise généralement des suspensions comportant de 1.10¹⁰ à 5.10¹² microorganismes pour 10 litres d'eau et éventuellement de 10 à 20 g/l d'un sel comestible et jusqu'à 1 g/l d'un acide organique comestible.

Suite à l'ensemencement, l'article de charcuterie peut être étuvé d'une manière classique, par exemple à des températures comprises entre 15°C et 35°C pendant 1 à 8 jours et à une humidité relative comprise entre 60 et 100%. L'étuvage permet l'acidification et la coloration de l'article de charcuterie et une première élimination d'eau.
Enfin l'article de charcuterie peut être séché, également de manière classique, par exemple à des températures comprises entre 10 et 15°C pendant une semaine à trois mois et à une humidité relative comprise entre 70 et 80%. Le séchage permet de compléter l'élimination de l'eau. C'est au cours du séchage que se développe principalement la fleur de surface. Outre les avantages mentionnés ci-dessus les microorganismes selon l'invention permettent de diminuer notablement le temps de séchage.

Dans le cas où ledit article de charcuterie est une saucisse ou un saucisson, si la peau est un menu, on ensemence de préférence avec une dispersion comportant essentiellement un microorganisme selon l'invention, tel Debaryomyces hansenii CBS 291.90. Si la peau est constituée par un chaudin ou un boyau artificiel on préfère alors utiliser une association selon l'invention.

Les exemples suivants ont pour but d'illustrer la présente invention.

### EXEMPLE 1 : préparation d'une saucisse sèche par douchage

a) pour préparer la mêlée, on hache dans un broyeur à viande :

| | |
|---|---|
| - gras (bardière) | 30% en poids |
| - maigre (épaule de porc) | 70% en poids |

dans lesquels on a rajouté :

| | |
|---|---|
| - NaCl | 30 g/Kg |
| - Nitrate de potassium | 0,05 g/Kg |
| - dextrose | 3 g/Kg |
| - saccharose | 3 g/Kg |
| - ail | 13 g/Kg |
| - ferments de maturation (Lactobacillus et Staphylococcus) | |

b) on embosse la mêlée dans un menu ; la mêlée est à une température de 0°C.
c) Ensemencement de la fleur de surface :
On procède à un ensemencement de la mêlée embossée par douchage au moyen d'une dispersion comportant :
- 10 l d'eau potable
- 1.10¹⁰ cellules de Debaryomyces hansenii CBS 291.90 par litre d'eau potable
- 10 g/l de NaCl

d) étuvage et séchage :
l'article de charcuterie ensemencé est placé dans une étuve :
- à 24°C pendant 8 heures
   (humidité relative de 95%)
- puis, on diminue la température régulièrement jusqu'à 15°C, en 40 heures
- enfin on sèche l'article de charcuterie dans un séchoir, pendant 10 jours à 12°C (humidité relative 76%)

La saucisse ainsi produite est sèche au toucher, et ne présente pas de tache de couleur, ce qui montre l'absence de contaminants, même trois mois après sa préparation.
La population bactérienne présente sur la peau de la saucisse a été comptée. Les résultats obtenus figurent dans le Tableau I ci-dessous. Ces résultats sont exprimés en nombre de cellules par cm² de surface de la peau de l'article de charcuterie.

### EXEMPLE 2 (comparatif)

On procède comme dans l'exemple 1 mais on douche la mêlée embossée au moyen d'une dispersion comportant Penicillium nalgiovensis PNT 1 commercialisée par la société Lacto Labo à la place de Debaryomyces hansenii CBS 291.90
Les saucisses ainsi préparées doivent être séchées pendant 17 jours. Elles sont poisseuses et présentent des taches de couleur bleue. Le comptage de la population bactérienne figure dans le Tableau I ci-dessous.

### EXEMPLE 3 : préparation d'une saucisse sèche par trempage

On procède comme dans l'exemple 1, mais on trempe la mêlée embossée au lieu de la doucher. Le trempage se fait dans la même dispersion que celle utilisée pour le douchage.
Les saucisses sèches obtenues présentent les mêmes caractéristiques que celles de l'exemple 1.

### EXEMPLE 4 (comparatif)

On prépare des saucisses sèches comme dans l'exemple 3, mais la dispersion comporte des Penicillium nalgiovensis à la place de Debaryomyces hansenii CBS 291.90. Ces saucisses sont poisseuses et comportent des taches vertes à leur surface.

### EXEMPLE 5 : préparation d'un saucisson sec

a) Pour préparer la mêlée, on hache dans un hachoir à viande :

| | |
|---|---|
| - gras (bardière) | 40% en poids |
| - maigre (épaule de porc ou cheval) | 60% en poids |

dans lesquels on rajoute :

| | |
|---|---|
| - NaCl | 28 g/Kg |
| - Nitrate de potassium | 0,055 g/Kg |
| - dextrose | 5 g/Kg |
| - lactose | 2,4 g/Kg |
| - ail | 3 g/Kg |
| - ferments de maturation (Pediococcus et Staphylococcus) | |

b) On embosse la mêlée dans un boyau cellulosique.
c) Ensemencement de la fleur de surface
On procède à un ensemencement de la mêlée embossée par douchage au moyen d'une dispersion comportant :
- 10 l d'eau potable
- 1.10¹⁰ cellules de Debaryomyces hansenii CBS 291.90 par litre d'eau potable
- 10 g/l de NaCl
- 0,1 g/l d'acide lactique

d) étuvage et séchage

L'article de charcuterie ensemencé est placé dans une étuve pendant 40 heures à 24°C puis dans un séchoir pendant 4 semaines à 12°C à une humidité relative de 78%.

Le saucisson sec ainsi obtenu, présente une fleur de surface poudreuse, rase, adhérente et de couleur crème. Il n'y a pas de tache de couleur, même après trois mois de conservation à l'air ambiant.

### EXEMPLE 6 (comparatif)

On procède comme dans l'exemple 5 mais le douchage se fait, au moyen d'une dispersion comportant 10 l d'eau dans laquelle est mis en suspension un mélange comportant 1.10¹⁰ spores de Penicillium nalgiovensis et 5.10¹⁰ cellules d'une souche classique (ne rentrant pas dans le cadre de la présente invention) de Debaryomyces hansenii. Un tel mélange est commercilaisé par la société Lacto Labo sous la marque LEM. Ladite dispersion comporte également 10 g/l de NaCl.

Le saucisson obtenu présente une fleur de surface épaisse, irrégulière et dont l'aspect est poisseux.
Le saucisson présente des taches bleues.

**Tableau I**

| Population/cm² de surface | Fleur de surface Debaryomyces hansenii CBS 291.90 | constituée par Penicillium nalgiovensis PNT 1 |
|---|---|---|
| Coliformes | moins de 10 | moins de 10 |
| Entérocoques | 70 | 750 |
| Bacteries psychrotrophes | moins de 10 | 600 |
| Micrococcaceae | 24.10⁵ | 18.10⁶ |

## Revendications

1. Levure de l'espèce Debaryomyces hansénii choisie parmi la souche CBS 291.90 ou parmi ceux de ses mutants ou recombinants qui présentent la propriété d'inhiber la formation de flore contaminante d'article de charcuterie et de former une fleur de surface sur la peau des articles de charcuterie.

2. Levure selon la revendication 1, caractérisée par le fait qu'elle est sous forme d'une culture pure.

3. Levure selon la revendication 1, caractérisée par le fait qu'elle est sous forme d'une culture lyophilisée.

4. Procédé de fabrication d'une levure selon l'une des revendications 1 à 3, caractérisé par le fait qu'il comporte les étapes d'introduction d'un inoculum de levure selon la revendication 1 dans un milieu de fermentation comportant une source carbonée et au moins une source azotée assimilable et de fermentation.

5. Application d'une levure selon l'une des revendications 1 à 3 à la formation de fleur de surface sur des peaux d'articles de charcuterie et pour inhiber la formation de la flore contaminant ladite fleur de surface.

6. Application selon la revendication 5 caractérisée en ce que ladite flore contaminante comporte ou est essentiellement constituée de moisissures.

7. Application selon l'une des revendications 5 et 6 caractérisée en ce que l'inhibition de la formation de la flore contaminante est observée pendant au moins un mois, et généralement plus de trois mois après un ensemencement de ladite levure sur la peau d'un article de charcuterie.

8. Association d'une levure selon l'une des revendications 1 à 3 avec au moins un microorganisme autre adapté pour la formation d'une fleur de surface sur la peau d'un article de charcuterie.

9. Association selon la revendication 8 caractérisée en ce que ledit microorganisme autre est une moisissure, de préférence une moisissure appartenant au genre Penicillium et plus préférentiellement aux espèces Penicillium nalgiovensis, Penicillium chrysogenum et Penicillium candidum.

10. Utilisation d'une levure selon l'une des revendications 1 à 3 ou d'une association selon l'une des revendications 8 et 9 pour la fabrication d'articles de charcuterie et de préférence d'articles de charcuterie recouverts de peau.

11. Procédé de fabrication d'articles de charcuterie caractérisé en ce qu'on ensemence notamment en trempant, douchant ou pulvérisant un article de charcuterie premier dans/ou au moyen d'une suspension aqueuse d'une levure selon l'une des revendications 1 à 3 ou d'une association selon l'une des revendications 8 et 9, puis éventuellement on étuve et on sèche ledit article de charcuterie.

12. Procédé selon la revendication 11 caractérisé en ce que ladite suspension aqueuse comporte en outre un sel comestible tel le chlorure de sodium et/ou un acide organique comestible tel l'acide lactique.

## Claims

1. A yeast of the species Debaryomyces hansenii selected from the CBS 291.90 strain or from those of its mutants or recombinants with the property of inhibiting the formation of contaminating flora on a prepared meat article and of forming a surface bloom on the skin of prepared meat articles.

2. Yeast according to claim 1, characterized in that it is in the form of a pure culture.

3. Yeast according to claim 1, characterized in that it is in the form of a freeze-dried culture.

4. A process for producing a yeast according to any one of claims 1 to 3, characterized in that it comprises the steps of introducing a yeast inoculum according to claim 1 into a fermentation medium comprising a carbon source and an assimilable nitrogen source, and fermenting.

5. Application of a yeast according to any one of claims 1 to 3 to forming a surface bloom on the skin of prepared meat articles and to inhibit the formation of contaminating flora on said surface bloom.

6. Application according to claim 5, characterized in that said contaminating flora comprises or is essentially constituted by moulds.

7. Application according to claim 5 or claim 6, characterized in that inhibition of contaminating flora formation is observed for at least one month, generally more than three months after seeding said yeast on the skin of a prepared meat article.

8. Association of a yeast according to any one of claims 1 to 3 with at least one other microorganism adapted for the formation of a surface bloom on the skin of a prepared meat article.

9. Association according to claim 8, characterized in that said other micro-organism is a mould, preferably a mould of the genus Penicillium and more preferably the species Penicillium nalgiovensis, Penicillium chrysogenum and Penicillium candidum.

10. Use of a yeast according to any one of claims 1 to 3 or of an association according to any one of claims 8 or 9 for the production of prepared meat articles, preferably prepared meat articles covered with a skin.

11. A process for producing prepared meat articles, characterized in that seeding is carried out, in particular by immersion, sprinkling or spraying of a starting prepared meat article in/or using an aqueous suspension of a yeast according to any one of claims 1 to 3 or an association according to claim 8 or claim 9, then optionally heating and then drying said prepared meat article.

12. A process according to claim 11, characterized in that said aqueous suspension further comprises an edible salt such as sodium chloride and /or an edible organic acid such as lactic acid.

## Patentansprüche

1. Hefe der Spezies Debaryomyces hansenii, ausgewählt aus dem Stamm CBS 291.90 oder unter jenen ihrer Mutanten oder Rekombinanten, die die Eigenschaft aufweisen, die Bildung von kontaminierender Flora auf Fleisch- und Wurstwaren zu inhibieren und einen Oberflächenschimmel auf der Haut der Fleisch- und Wurstwaren zu bilden.

2. Hefe nach Anspruch 1, dadurch gekennzeichnet, daß sie in Form einer Reinkultur vorliegt.

3. Hefe nach Anspruch 1, dadurch gekennzeichnet, daß sie in Form einer lyophilisierten Kultur vorliegt.

4. Verfahren zur Herstellung einer Hefe nach einem der Ansprüche 1 bis 3, dadurch gekennzeichnet, daß es die Schritte eines Hinzugebens eines Inoculums von Hefe nach Anspruch 1 zu einem Fermentationsmedium, umfassend eine kohlenstoffhaltige Quelle und mindestens eine assimilierbare stickstoffhaltige Quelle, und einer Fermentation umfaßt.

5. Anwendung einer Hefe nach einem der Ansprüche 1 bis 3 für die Bildung von Oberflächenschimmel auf der Haut von Fleisch- und Wurstwaren und zum Inhibieren der Bildung von den Oberflächenschimmel kontaminierender Flora.

6. Anwendung nach Anspruch 5, dadurch gekennzeichnet, daß die kontaminierende Flora Schimmelpilze umfaßt oder im wesentlichen daraus gebildet wird.

7. Anwendung nach einem der Ansprüche 5 und 6, dadurch gekennzeichnet, daß die Inhibition der Bildung der kontaminierenden Flora während mindestens einem Monat und im allgemeinen mehr als drei Monate nach einem Animpfen der Hefe auf die Haut einer Fleisch- oder Wurstware beobachtet wird.

8. Zusammenstellung einer Hefe nach einem der Ansprüche 1 bis 3 mit mindestens einem anderen Mikroorganismus, der für die Bildung eines Oberflächenschimmels auf der Haut einer Fleisch- oder Wurstware angepaßt ist.

9. Zusammenstellung nach Anspruch 8, dadurch gekennzeichnet, daß der andere Mikroorganismus ein Schimmelpilz, vorzugsweise ein Schimmelpilz, der aus der Gattung Penicillium und noch mehr bevorzugt aus den Spezies Penicillium nalgiovensis, Penicillium chrysogenum und Penicillium candidum stammt, ist.

10. Verwendung einer Hefe nach einem der Ansprüche 1 bis 3 oder einer Zusammenstellung nach einem der Ansprüche 8 und 9 für die Herstellung von Fleisch- und Wurstwaren und vorzugsweise von vollständig mit Haut bedeckten Fleisch- und Wurstwaren.

11. Verfahren zur Herstellung von Fleisch- und Wurstwaren, dadurch gekennzeichnet, daß man insbesondere durch Eintauchen, Abduschen oder Zerstäuben eine erste Fleisch- oder Wurstware in oder mittels einer wäßrigen Suspension einer Hefe nach einem der Ansprüche 1 bis 3 oder einer Zusammenstellung nach einem der Ansprüche 8 und 9 animpft, dann gegebenenfalls inkubiert und die Fleisch -oder Wurstware trocknet.

12. Verfahren nach Anspruch 11, dadurch gekennzeichnet, daß die wäßrige Suspension darüber hinaus ein eßbares Salz, wie Natriumchlorid, und/oder eine eßbare organische Säure, wie Milchsäure, umfaßt.
